Europäisches Patentamt

**European Patent Office** (11) Publication number: **0 011 663**

Office européen des brevets **B1**

(12) **EUROPEAN PATENT SPECIFICATION**

(45) Date of publication of patent specification: **08.12.82** (51) Int. Cl.³: **A 61 K 7/16, A 61 K 7/22**

(21) Application number: **78300712.3**

(22) Date of filing: **01.12.78**

(54) Toilet and dental preparations and their use for oral and dental hygiene.

| | |
|---|---|
| (43) Date of publication of application:<br>**11.06.80 Bulletin 80/12** | (73) Proprietor: **UNILEVER PLC**<br>**Unilever House Blackfriars  P O Box 68**<br>**London EC4P 4BQ (GB)**<br>㊽ **GB** |
| (45) Publication of the grant of the patent:<br>**08.12.82 Bulletin 82/49** | (73) Proprietor: **UNILEVER NV**<br>**Burgemeester s'Jacobplein 1 P.O. Box 760**<br>**NL-3000 DK  Rotterdam (NL)**<br>㊽ **BE CH DE FR IT LU NL SE** |
| (84) Designated Contracting States:<br>**BE CH DE FR GB IT LU NL SE** | (72) Inventor: **Ritchey, Thomas William**<br>**206 Somerset Road**<br>**Norwood New Jersey (US)** |
| (56) References cited:<br>**BE - A - 569 397**<br>**DE - A - 2 310 771**<br>**DE - A - 2 310 772**<br>**DE - C - 442 857**<br>**FR - A - 2 282 861**<br>**GB - A - 654 473**<br>**GB - A - 1 290 627**<br>**US - A - 1 433 979**<br>**US - A - 3 201 316**<br>**US - A - 3 761 583**<br>**US - A - 3 772 431**<br>**US - A - 3 914 406**<br>**US - A - 4 105 759** | (74) Representative: **Stancliffe, Terence Christopher**<br>**et al,**<br>**Unilever PLC, Patent Division PO Box 31 Salisbury**<br>**Square House Salisbury Square**<br>**London EC4P 4AN (GB)** |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# 0 011 663

Toilet and dental preparations and their use for oral and dental hygiene

The field to which this invention relates is that of oral and dental hygiene, and of toilet and dental preparations for such hygiene purposes.

In particular the invention relates to oral products (such as dentifrices and mouthwashes) which have anti-plaque activity, i.e. those which are effective in retarding the formation of oral plaque in the mouths of users.

There are already numerous prior art disclosures relating to substances with anti-plaque activity. Among these are disclosures of the use of aliphatic amines, e.g. decylamine, dodecylamine, tetradecylamine and hexadecylamine, as showing such activity in tests carried out *in vitro*. Tetradecylamine was studied separately by R. M. King, *J. Dental Res.* (1951), *30*, 399—402, in trials using tetradecylamine in medicinal paraffin.

US Patent No. 3,943,267 (Randol) discloses use of heavy metals in treatment of tooth enamel by a process including acid-etching and heavy-metal deposition on the etched surfaces: the metals used include zinc, iron, chromium, nickel, lead, cobalt, cadmium, copper, platinum, gold and silver; and the process requires conversion to metal sulphides in vivo to resist decay.

There are also disclosures of certain metal compounds as ingredients in such oral products as dentifrices and mouthwashes, e.g. zinc ions (US Patent No. 4 022 880 (Vinson & Cancro) and UK Patent No. 1 373 001 (Unilever) and US Patent No. 3 888 976 (Mlkvy & Tucci)).

We consider it desirable to find ways of increasing the activities of active ingredients present in anti-plaque oral hygiene compositions, so that for a given effect less of the active ingredients is required.

This invention is based on the finding that the anti-plaque activity of oral products containing tetradecylamine can be enhanced by the presence of compounds of certain metals to an extent greater than that attributable to the mere presence of the metal compounds themselves: i.e. the effect is what has been called synergistic. A similar effect was sought, but not found, in mixtures in which dodecylamine or hexadecylamine was present instead of the tetradecylamine as an anti-plaque agent.

Accordingly, by this invention, there is provided an oral composition, such as a dentifrice or a mouthwash, comprising:

(a) tetradecylamine,

(b) at least one water-soluble salt of zinc, aluminium, copper, iron, nickel, cobalt and/or tin producing positively charged metal ions in aqueous solution; and

(c) a base acceptable for oral use and compatible with components (a) and (b): in which composition the concentration of the tetradecylamine component (a) is in the range 0.005—0.05 molar and component (b) comprises a zinc or an aluminium salt at a concentration in the range 0.001—0.05 molar, or a copper, iron, nickel, cobalt or tin salt at a concentration in the range 0.0005—0.015 molar.

The oral compositions of this invention can take the form of mouthwashes, toothpastes, tooth powders, lozenges, dental creams and/or chewing gum. They enable plaque to be controlled when they are applied to the mouths of users, and are hence useful in the prophylaxis of tooth decay and periodontal diseases. The concentration of the tetradecylamine component (a) in the composition will preferably, for many purposes, be chosen within the range 0.005—0.05 molar.

A plurality of compounds of one or a plurality of metals as specified at (b) can be present, if desired. According to the Seventh Issue of the Merck Index, aluminium and zinc have an $LD_{50}$ orally in rats of approximately 3—4 g/kg body weight; copper, iron, nickel, cobalt have $LD_{50}$ (orally in rats) in the range 0.3—0.9 g/kg body weight, and tin has an $LD_{50}$ (orally in rats) in the range about 0.05 g/kg body weight. The synergism observed between the active ingredients (a) and (b) in the compositions of this invention has the result that plaque may be controlled with relative ease by using substantially non-toxic amounts of the metal compound with a substantially non-toxic amine.

The essence of the invention consists, thus, of a synergistic combination with greater antiplaque activity that the simple additive effect of both of the constituent compounds. Tetradecylamine when used alone exhibits good antiplaque activity but when coupled with the metal compound component (b) of this invention, gives significant additional activity. In addition to their synergistic activity, the metals were chosen for their solubility, low astringency and low toxicity.

While not wishing to be bound by the following theories, it is postulated first that the amine facilitates the penetration of the bacterial cell wall by the germicidal metal. Charged metals are normally excluded from inside microorganisms by a lipid-protein cell wall. The lipophylic tetradecylamine may solubilise the cell wall or mask the charge of the metal to allow passage of the particle into the bacterial cell. An increased death of microorganisms will be manifested in a reduction of plaque growth and its accumulation. Several antibiotics, including the sulfa drugs, have been shown to work synergistically with metals. While antibacterial agents alone will kill or retard the growth of plaque bacteria, neither the metal ions nor the tetradecylamine of this invention can easily be used at levels permissible in the oral cavity which produce the technical effect possible with the two in combination. Additionally, as a second postulate, the level of antiplaque activity may be increased

2

because the synergistic combination reduces the surface energy and surface tension of the tooth pellicle. The pellicle may be covered by a low surface tension monolayer of tetradecylamine and positively charged metal ions. In effect, this lower surface tension may reduce the affinity of plaque to the dentition. Further, this monolayer may reduce the incidence of caries by preventing the transport of ions from the enamel surface.

The latter theory, regarding lowering of surface tension, is favoured since the antiplaque compositions of the instant invention failed to demonstrate synergistic germicidal activity in bacterial cultures. In addition, the critical micellar concentration (CMC) of tetradecylamine was found to be lowered in combination with selected metals. Bass, Dillingham and Powers (J. Dent. Res. *54*: 968— 971, (1975)) showed that lowering the CMC in a series of aliphatic amines increased the antiplaque activity. The CMC of other amines was not lowered in combination with zinc. It was found that lowering the CMC paralleled the finding of synergistic plaque reduction of the present invention. Aluminium and/or zinc metals are thought to lower the CMC of tetradecylamine by forming ion pairs. This theory was further confirmed when it was found that the addition of $CO_2$ to tetradecylamine increased antiplaque activity while lowering the CMC. The final evidence was offered when it was found that during formulation, if non-ionic detergents such as those sold under the trade marks Brij, Tween or Triton were employed, the CMC of tetradecylamine in combination with selected metals was raised to that concentration expected for tetradecylamine alone, while the synergistic plaque reductions were lost, leaving plaque reductions indicative of only tetradecylamine.

Dodecylamine, dimethyldodecylamine, hexadecylamine, decylamine, and N,N'-dimethyl-octadecylamine do not give synergistic plaque reductions. Only the primary aliphatic amine with a fourteen carbon chain length acted synergistically with zinc or aluminium.

Under the conditions of the tests described below, the minimum amount of the metal compound or mixtures of compounds necessary to result in control of plaque is generally about 0.001 molar for aluminium and zinc, and 0.0005 molar for copper, nickel, iron, cobalt and tin, at the site of formation of the plaque. While there seems to be no maximum effective concentration, an amount significantly higher than about 0.05 molar aluminium and zinc, and 0.015 molar copper, nickel, iron, cobalt and tin, may be difficult to formulate in an organoleptically acceptable mouthwash. The particularly preferred metal concentration (molar) is about 0.018 aluminium, 0.020 for zinc, 0.0037 iron, 0.0042 nickel, 0.0042 cobalt, 0.0041 copper, and 0.0019 tin in combination with tetradecylamine in a mouthrinse for application to plaque. The maximum concentration which can be utilised is properly determined by product parameters such as astringency and formulation compatibilities. The preferred pH of a mouthrinse is in the range pH 3—5, particularly preferred is about pH 4.1 for aluminium, pH 4.3 for zinc, pH 5 for copper, iron, nickel and cobalt, and pH 3 for tin, to deliver sufficient metal ions to the site of plaque formation.

The concentration of metal containing compound combined with a vehicle and the tetradecylamine to form the compositions of this invention is not critical and may vary within an organoleptically and toxicologically acceptable limit so long as the amount of compound is sufficient to result in the concentration of 0.0005 to 0.05 molar at the site of formation of the plaque, preferably 0.01—0.05 molar in the case of aluminium and zinc, and 0.0005—0.015 molar in the case of copper, iron, nickel, cobalt, and tin.

The optimum amount of the tetradecylamine necessary to result in a control of plaque when combined with the metals of the instant invention is generally about 0.005 molar in a rinse. While there is no maximum effective concentration, amounts significantly higher than about 0.05 molar will be difficult to formulate in an organoleptically and toxicologically acceptable mouthwash or manner.

The maximum concentration of tetradecylamine which can be utilised is thus properly determined by product parameters as listed above in relation to the maximum amount of metal which can be used.

Without tetradecylamine the high level of the metal which would be necessary to observe significant benefit may be too astringent and/or toxic for normal therapeutic use. Furthermore, high levels of plaque reduction with a metal have never been demonstrated in a clinical study. In addition, high levels of metals are difficult to formulate into a product acceptable to the consumer and conformable with legislation applicable to oral products.

Tetradecylamine without a metal does not attain the desired plaque reductions. While higher levels of this amine confer additional benefit, its bitter taste, greater toxicity, problems of solubility and compatibility with the vehicle provide practical limits of increasing its concentration.

This invention suggests the use of the highest possible level of tetradecylamine which is non-toxic and suitable for formulations. The addition of the metal to the amine does not markedly affect the toxicity or acceptability of the amine. By using the synergistic mixtures of this invention advantage is taken not only of their co-action, but also of the fact that low levels of each can be employed.

In one embodiment, the invention comprises a water-alcohol soluble tetradecylamine/metal mouthwash (oral rinse) which reduces dental plaque. In a further embodiment, the combination of the invention may be used in a dentifrice. The essential components of the invention are an orally acceptable medium, which may be for example, water and alcohol, and the antiplaque mixture. The term "orally acceptable medium" applies to any suitable carrier medium for the antiplaque mixture;

# 0 011 663

such a medium is selected to be harmless to the oral cavity and is not meant to be intentionally swallowed; however the medium is, of course, harmless in an amount accidentally ingested during use.

The compositions of the instant invention may be utilised with a variety of orally compatible agents such as mouthwashes, toothpastes, dentifrices, tooth powders, lozenges, and chewing gum as well as any compatible vehicle for applying the synergistic combination at the specific site of plaque formation. Such formulations are generally prepared in accordance with the art-recognised practice.

In mouthwash formulations, for example, the medium includes typically an essentially aqueous solution of alcohol, glycerine or sorbitol. In some mouthwash formulations it is not essential to use any of these materials although they do help to solubilise certain flavour oils. A suitable mouthrinse, for example, contains about 0.02 molar tetradecylamine and about 0.02 molar zinc or aluminium, or 0.004 molar copper, iron, cobalt, nickel, or 0.002 tin, in a medium consisting essentially of 75%—95% water and 5—25% ethanol. Other common additives that do not affect synergism may also be present.

In toothpastes and tooth powder formulations, the essential ingredient other than tetradecylamine and the aluminium and/or zinc compound of this invention is any suitable dental abrasive compatible with components (a) and (b) of the composition. It is recommended that the abrasives used in the dentifrice formulation of the present invention provide a final composition which has an acceptable dentin abrasion value. Suitable dental abrasive substances include finely divided particles of appropriate size, hardness and composition for dentifrice abrasives.

Toothpastes and tooth powder formulations also commonly contain a soap or synthetic surface active agent. It is essential in these formulations as well as mouthwash formulations to provide sufficient foaming action to satisfy a market consumer preference for this property. A preferred material for dentifrices is sodium lauryl sulfate. However, many other surface active agents can be used so long as they are compatible, i.e. they do not interfere with the activity of the compound synergistic interaction between the tetradecylamine and the metal compound. It has however been noted that in some compositions nonionic detergents did inactivate the synergism. The practitioner in the art will be able to test for this effect by the methods referred to herein and avoid such inactivation.

In addition, the toothpaste formulation will frequently contain humectants sufficient to provide smooth texture and flowability. Glycerine and sorbitol are preferred for this purpose together with suitable amounts of water, ethyl alcohol, glucose, and mannitol.

Lastly, the toothpaste formulation generally contains selected binding agents. These also should be compatible with the synergistic combination as well as with the other toothpaste components. For example, cellulose ethers are one type of preferred binder.

A chewing gum medium normally comprises a gum base and common flavouring materials used in the field. The flavouring materials are present at a level of about 0.01—2.0% of the final chewing gum composition. The base is a chewable plastic gum material such as natural rubber, chickle, polyvinyl acetate, ester gum, coumarone resin, and paraffin wax. The gum base is typically made from a mixture of two or more plastic gum materials to achieve a preferred degree of plasticity for chewing. Optionally, a binder or a softener may be used as well as sweetening agents.

Lozenges may be made containing the synergistic combination with a suitable binder.

The invention is further illustrated hereinbelow by a number of Tests and Examples.

An *in vitro* test was used in the Tests described herein for examining and effects of chemotherapeutic agents on plaque, and was carried out in accordance with the test reported in the *Journal of Dental Research*, Vol. *55*, February 1976, page B286, by R. T. Evans, P. J. Baker, R. A. Coburn and R. J. Genco. An assay system for producing artifical plaque was used to screen antiplaque agents rapidly under conditions which simulated those found in the oral cavity. Evans *et al*. found that effective dosages in the *in vitro* assay correlated with results of previously published clinical studies. The assay gives reproducible, quantitative results and has the ability to distinguish between antibacterial agents which are clinically effective or non-effective as plaque inhibitors.

The *in vitro* plaque was formed at 37°C on uniformly-sized aluminium plummets which were first coated with saliva. The plummets were than placed in a growth medium inoculated with clinical plaque samples. After seven hours the plummets were suspended overnight in a 25% saliva mixture. On the second day, the plummets were immersed in a 50% saliva—50% test compound mixture for one minute, placed in the growth medium for seven hours, retreated with the test mixture and suspended in 25% saliva overnight. The plummets were treated again on the third day and incubated in the growth medium for three hours. The plaques were removed from the plummets by sonication into approximately six millilitres of buffer and quantitated by optical density (O.D.) in a Beckman DU spectrophotometer at 570 nm.

A test compound showed antiplaque activity if the O.D. (plaque mass) was reduced from the control plaques treated with water. Positive controls were included to determine relative activity. Comparisons of antiplaque activity were then made within a given experiment. At least five replicas per compound were examined.

The following Tests and Examples will more fully illustrate the embodiments of this invention. All parts and proportions referred to herein and in the appended claims are by weight unless otherwise noted.

4

**0 011 663**

Test 1

The effects of $H_2O$, 0.04% cetyl pyridinium chloride (CPC), 0.060% tetradecylamine (TDA); 0.30% $ZnCl_2$ and several concentrations of zinc salt plus tetradecylamine are examined, by the artificial plaque method previously described, all with 15% ethanol, at pH 4.1. The results are as follows in Table I.

TABLE I

| Active | % Wt/ Vol. | | Average optical density | Standard deviation | % Reduction |
|---|---|---|---|---|---|
| $H_2O$ (control) | — | | .704 | .028 | — |
| CPC alone | .040 | | .515 | .014 | 26.9 |
| TDA alone | .060 | | .571 | .035 | 18.8 |
| $ZnCl_2$ alone | .30 | | .766 | .022 | −8.0* |
| $ZnCl_2$ plus TDA | .10 .060 | > | .451 | .012 | 35.9 |
| $ZnCl_2$ plus TDA | .20 .060 | > | .436 | .041 | 38.0 |
| $ZnCl_2$ TDA | .30 .060 | > | .394 | .022 | 44.0 |

*Weight gain

At the concentrations of metal salt alone used in this test, there is often a small increase in plaque weight with the *in vitro* assay. Clincial data suggest however that zinc salt alone provides about 5% reduction in plaque.

Tetradecylamine alone was slightly less effective than the quaternary ammonium germicide, CPC. This level of TDA is usable in an oral product for human use without creating any problems due to toxicity. The results shown in Table I clearly show that the combination of $ZnCl_2$ and tetradecylamine produced a greater anti-plaque effect than the sum of the effects produced by either ingredient alone, i.e. a synergistic effect.

The dose-response curve of tetradecylamine indicates the necessity of increasing the concentration from 0.060% to 0.20% in order to raise the plaque reduction from 18% to 44% when a metal is not present. This higher level would be expected to be more toxic and more bitter than lower levels. The synergistic effect of combining the use of zinc chloride with that of tetradecylamine, as disclosed herein, allows such drawbacks to be mitigated.

Test 2

To test higher levels of tetradecylamine, the artificial plaque treatment previously described is eliminated on the third day to simulate infrequent use of the antiplaque products. The results of tetradecylamine in combination with salts of zinc or aluminium are listed in Table II.

TABLE II

| Active | % Wt/ Vol. | | Average optical density | Deviation | % Reduction |
|---|---|---|---|---|---|
| $H_2O$ (control) | — | | 1.113 | .018 | — |
| TDA alone | .120 | | .882 | .076 | 20.7 |
| $AlCl_3 . 6H_2O$ alone | 1.00 | | 1.218 | .026 | −9.4* |
| $AlCl_3 . 6H_2O$ alone | .250 | | 1.080 | .045 | 3.0 |
| $AlCl_3 . 6H_2O$ plus TDA | 1.00 .120 | > | .371 | .034 | 66.6 |
| $AlCl_3 . 6H_2O$ plus TDA | .50 .120 | > | .161 | .012 | 88.5 |
| $AlCl_3 . 6H_2O$ plus TDA | .250 .120 | > | .150 | .011 | 86.5 |
| $ZnCl_2$ plus TDA | .20 .120 | > | .456 | .026 | 59.0 |

*Weight gain

These data show that even low amounts of aluminium salt in combination with tetradecylamine cause the synergistic reduction of plaque. When compared with zinc salt and tetradecylamine, equimolar amounts of aluminium salt and tetradecylamine give statistically greater reductions. In

5

addition, aluminium salt is less astringent and may be less toxic, thus, aluminium salt plus tetradecylamine may be more useful in a therapeutic product.

It is postulated that the weight gains observed with the metal salts alone are the result of the precipitation of salivary components onto the plummets. Even without regarding these as weight gains, both metal salt combinations with the amine give wide margins of synergistic reductions over the relatively low reduction seen for the tetradecylamine alone.

Cations such as hydrogen, sodium potassium, magnesium and calcium, have not been found to give synergistic plaque reductions by the *in vitro* assay. In addition, synergism has not been found associated with chloride, fluoride, nitrate, phosphate and sulphate in themselves.

Examples 1—2

A tooth powder and toothpasts are made up to the following specification:

### Example 1

| Tooth powder | % |
|---|---|
| Abrasive | 90.0 |
| Sodium lauryl sulfate | 3.0 |
| Hydrochloric acid | (to pH 3.8) |
| $Al_2(SO_4)_3$ | 3.0 |
| Tetradecylamine | 3.0 |
| Flavour | 1.0 |
| | 100.0% |

### Example 2

| Toothpaste | % |
|---|---|
| Particulate polishing agents | 10.00 |
| Humectant (sorbitol) | 40.00 |
| Sodium lauryl sulfate (21%) glycerine | 7.00 |
| Bodying agent (carboxymethyl cellulose) | 1.00 |
| Flavour and colour | 1.5 |
| Zinc chloride | 1.2 |
| Tetradecylamine | 0.3 |
| Hydrochloric acid | (to pH 4.4) |
| Water | Balance to 100.00% |

The above Example formulations 1—2 both incorporate synergistic mixtures according to this invention. The specific metal salts used in the formulation are typical of the metal salts which can be used with tetradecylamine (TDA). The antiplaque effect of the formulations is greater than that accountable by the sum of the effects of either tetradecylamine or metal salts alone.

Test 3

Several separate experiments are conducted by the artificial plaque method previously described to demonstrate the synergistic plaque reductions associated with copper, iron, nickel and cobalt in combination with the tetradecylamine (TDA). The experiments are conducted in the same manner, in a 15% ethanol/water media at pH 5. The values are presented below in Table III.

### TABLE III

| Active | | % Wt./Vol. | % Reduction |
|---|---|---|---|
| TDA alone | | 0.06 | 19 |
| $Cu(NO_3)_2 . 3H_2O$ alone | | 0.10 | −10 |
| $Cu(NO_3)_2 . 3H_2O$ and TDA | > | 0.10 / 0.06 | 62 |
| $FeCL_3 . 6H_2O$ alone | | 0.10 | −38 |
| $FeCl_3 . 6H_2O$ and TDA | > | 0.10 / 0.06 | 49 |
| $NiCl_2 . 6H_2O$ alone | | 0.10 | −17 |
| $NiCl_2 . 6H_2O$ and TDA | > | 0.10 / 0.06 | 69 |
| $CoCl_2 . 6H_2O$ alone | | 0.10 | −25 |
| $CoCl_2 . 6H_2O$ and TDA | > | 0.10 / 0.06 | 59 |

The data show that the combination of these metals and tetradecylamine produced a synergistic effect in plaque reduction.

Similar comments apply to the interpretation of these test results as were made in connection with Tests 1 and 2 above.

Examples 3—6

A mouthwash and toothpastes are made up to the following specifications:

### Example 3

| Mouthwash | % |
|---|---|
| $FeCl_3 . 6H_2O$ | 0.07 |
| Tetradecylamine | 0.3 |
| Flavour | 0.15 |
| Humectant (Fructose) | 80.00 |
| Saccharin | 0.02 |
| Colouring | 0.22 |
| Hydrochloric acid | (to pH 5) |
| Ethanol | 15.00 |
| Water | Balance to 100 % |

### Example 4

| Toothpaste | % |
|---|---|
| Abrasive | 10.00 |
| $NiCl_2 . 6H_2O$ | 0.05 |
| $CoCl_2 . 6H_2O$ | 0.05 |
| Refined extract of carrageenan | 0.35 |
| Tetradecylamine | 0.25 |
| Bodying agent (Syloid 44) (Trade Mark) | 9.00 |
| Saccharin | 0.20 |
| Glycerine (95%) | 50.00 |
| Hydrochloric acid | (to pH 5) |
| 21% sodium lauryl sulfate in glycerine | 7.00 |
| Colouring and flavour | 1.32 |
| Water | Balance to 100% |

### Example 5

| Toothpaste | % |
|---|---|
| Abrasive | 15.00 |
| $Cu(NO_3)_2 . 3H_2O$ | 0.4 |
| Powdered polyethylene[1] | 5.00 |
| Tetradecylamine | 0.2 |
| Carboxymethyl cellulose | 0.80 |
| Glycerine | 65.00 |
| Saccharin | 0.20 |
| Flavour | 1.30 |
| Colouring | 0.25 |
| Foaming agent | 0.63 |
| Hydrochloric acid | (to pH 5) |
| Water | Balance to 100% |

[1]The polyethylene is a high density polyethylene powder having an average particle size of about 8—9 microns.

**0 011 663**

### Example 6

| Toothpaste | | % |
|---|---|---|
| Abrasive | | 17.00 |
| $FeCl_3 . 6H_2O$ | | 0.6 |
| Polyethylene powder[1] | | 6.00 |
| Tetradecylamine | | 0.2 |
| Carboxymethyl cellulose | | 0.80 |
| Saccharin | | 0.35 |
| Glycerine | | 55.00 |
| Flavour | | 1.30 |
| Foaming agent (sodium lauryl sulfate) | | 1.47 |
| Colour | | 0.25 |
| Acetic acid | | (pH to 5.5) |
| Water | Balance to | 100% |

[1]The polyethylene is a high density polyethylene powder having an average particle size of about 8—9 microns

The above Examples 3—6 incorporate synergistic mixtures according to this invention. The antiplaque effect of the formulations is greater than that accountable by the sum of the effects of either tetradecylamine or metal salts alone.

Test 4

The effects of 0.060% tetradecylamine (TDA) 0.05 $SnCl_4$, and the combination of $SnCl_4$ and TDA at these concentrations are examined by the artificial plaque method previously described, all with 15% ethanol, at pH 2.6. The results are as follows in Table IV.

TABLE IV

| Active | | % Wt/Vol. | % Reduction |
|---|---|---|---|
| TDA alone | | 0.06 | 19 |
| $SnCl_4 . 5H_2O$ alone | | 0.05 | —8* |
| { $SnCl_4 . 5H_2O$ plus | > | 0.06 | 50 |
| { TDA | | 0.06 | |

*Weight gain

At these metal concentrations, there was often a small increase in plaque weight with the *in vitro* assay. Clinical data suggest that tin salt alone provides slight reductions in plaque.

As with Tests 1—3, it is postulated that the weight gains observed with the metals alone are the result of the precipitation of salivary components onto the plummets. Even ignoring these weight gains, the metal/amine combinations gave wide margins of synergistic reductions over the relative low reduction seen for the tetradecylamine alone.

Cations such as hydrogen, sodium, potassium, magnesium, and calcium, did not give synergistic plaque reductions by the *in vitro* assay. In addition, synergism was not found associated with chloride, fluoride, nitrate, phosphate and sulphate in themselves.

The combination of $SnCl_4$ and tetradecylamine clearly produced a synergistic effect.

The dose-response of tetradecylamine indicates the necessity of increasing the concentration from 0.060% to 0.20% in order to raise the plaque reduction from 18% to 44% when tin is not present. This higher level would be expected to be more toxic and more bitter than lower levels.

Examples 7—8

A mouthwash and toothpaste were made up to the following specifications:

### Example 7

| Mouthwash | | % |
|---|---|---|
| Ethanol | | 22.00 |
| Glycerol | | 12.00 |
| Flavour and Colour | | 0.90 |
| Hydrochloric acid | | (to pH 3) |
| $SnCl_4 . 5H_2O$ | | 0.03 |
| Tetradecylamine | | 0.01 |
| Water | Balance to | 100% |

**0 011 663**

Example 8

| Toothpaste | % |
|---|---|
| Abrasive | 13.00 |
| Binder | .30 |
| Sorbitol (70% solution) | 64.20 |
| Cab-O-Sil bodying agent | 5.00 |
| $SnCl_2$ | 0.15 |
| Tetradecylamine | 0.8 |
| 21% Sodium lauryl sulfate in glycerine | 7.00 |
| Hydrochloric acid | (to pH 3.5) |
| Flavour and colour | 3.00 |
| Water | Balance to 100% |

The above examples 7—8 incorporate synergistic mixtures according to this invention. The antiplaque effect of the formulations is greater than that accountable by the sum of the effects of either tetradecylamine or metal salts alone.

In summary, it appears that the presence of quantities of salts of the metals mentioned above which are in themselves insufficient to give more than a very small antiplaque effect (or which give none at all) can nevertheless result in a substantial increase in the anti-plaque effectiveness of tetradecylamine present in an oral product.

## Claims

1. An oral composition comprising
(a) tetradecylamine;
characterised in that it also comprises:
(b) at least one water-soluble salt of zinc, aluminium, copper, iron nickel, cobalt and/or tin producing positively charged metal ions in aqueous solution; and
(c) a base acceptable for oral use and compatible with components (a) and (b): in which composition the concentration of the tetradecylamine component (a) is in the range 0.005—0.05 molar and component (b) comprises a zinc or an aluminium salt at a concentration in the range 0.001—0.05 molar, or a copper, iron, nickel, cobalt or tin salt at a concentration in the range 0.0005—0.015 molar.

2. An oral composition according to Claim 1, in which component (b) comprises a salt of one of the following metals at about the molar concentration indicated as follows: aluminium or zinc at about 0.02; iron, nickel, cobalt, or copper at about 0.004; or tin at about 0.002.

3. An oral composition according to Claim 1 or Claim 2 in which the concentration of tetradecylamine is about 0.02 molar.

4. An oral composition according to any preceding claim in the form of a dentifrice such as a toothpaste or tooth powder; a dental cream; a mouthwash; a lozenge or a chewing gum.

5. An oral composition according to Claim 4, which is a dentifrice and contains a dental abrasive compatible with components (a) and (b) of the composition.

6. For use in dental hygiene, or for controlling oral plaque, or retarding its formation, or in the prophylaxis of tooth decay or periodontal disease, an oral composition as defined in any one of the preceding claims.

## Revendications

1. Composition orale comprenant (a) de la tétradécylamine; caractérisée en ce qu'elle comprend aussi: (b) au moins un sel, soluble dans l'eau, de zinc, aluminium, cuivre, fer, nickel, cobalt et/ou étain, produisant des ions positivement chargés en solution aqueuse; et (c) une base acceptable pour l'usage orale et compatible avec les composants (a) et (b); composition dans laquelle la concentration molaire du composant tétradécylamine (a) est comprise dans l'intervalle de 0,005 à 0,05 et le composant (b) comprend un sel de zinc ou d'aluminium dont la concentration molaire est comprise dans l'intervalle de 0,001—0,05, ou un sel de cuivre, fer, nickel, cobalt ou étain dont la concentration molaire est comprise dans l'intervalle de 0,0005—0,015.

2. Composition selon la revendication 1, caractérisée en ce que le composant (b) comprend un sel d'un des métaux suivants à la concentration molaire d'environ 0,02 pour l'aluminium ou le zinc; 0,004 pour le fer cobalt, nickel ou cuivre; ou 0,002 pour l'étain.

3. Composition suivant l'une des revendications 1 et 2, caractérisée en ce que la concentration molaire de la tétradécyllamine est d'environ 0,02.

4. Composition suivant l'une des revendications 1 à 3, caractérisée en ce qu'elle est sous forme d'un produit dentifrice, tel qu'une pâte ou une poudre dentifrice, une crème dentaire, un bain de bouche, des pastilles ou du chewing-gum.

5. Composition selon la revendication 4, caractérisée en ce qu'elle est un produit dentifrice et contient un abrasif dentaire compatible avec les composants (a) et (b).

6. Procédé d'hygiène dentaire, ou pour le contrôle de la plaque orale, ou pour retarder sa formation, ou de prophylaxie des caries dentaires ou de la maladie périodontale, caractérisé par l'emploi d'une composition selon l'une des revendications 1 à 5.

**Patentansprüche**

1. Orale Zusammensetzung enthaltend
   (a) Tetradecylamin;
dadurch gekennzeichnet, daß sie weiterhin enthält:
   (b) mindestens ein wasserlösliches Salz von Zink, Aluminium, Kupfer, Eisen, Nickel, Kobalt und/oder Zinn, welches positiv geladene Metallionen in wässriger Lösung bildet; und
   (c) eine Base annehmbar für orale Verwendung und verträglich mit den Komponenten (a) und (b): wobei die Konzentration der Tetradecylaminkomponente (a) im Bereich von 0,005—0,05 molar ist und die Komponente (b) ein Zink- oder Aluminiumsalz in einer Konzentration im Bereich von 0,001—0,05 molar oder ein Kupfer-, Eisen-, Nickel-, Kobalt- oder Zinnsalz in einer Konzentration im Bereich von 0,0005—0,015 molar ist.

2. Orale Zusammensetzung gemäß Anspruch 1, in welcher die Komponente (b) ein Salz einer der folgenden Metalle in der angegebenen Molarkonzentration ist: Aluminium oder Zink in etwa 0,02; Eisen, Nickel, Kobalt oder Kupfer in etwa 0,004 oder Zinn in etwa 0,002.

3. Orale Zusammensetzung gemäß Anspruch 1 oder 2, in welcher die Konzentration von Tetradecylamin etwa 0,02 molar ist.

4. Orale Zusammensetzung gemäß einem der vorhergehenden Ansprüche in Form eines Zahnputzmittels wie einer Zahnpaste oder einem Zahnpulver; einer Dentalcreme; einem Mundwaschmittel; einer Tablette oder einem Kaugummi.

5. Orale Zusammensetzung gemäß Anspruch 4 in Form eines Zahnputzmittels und enthaltend ein Zahnscheuermittel verträglich mit den Komponenten (a) und (b) der Zusammensetzung.

6. Zur Verwendung in der Dentalhygiene oder zur Kontrolle von oralem Plaque oder zur Verhinderung von dessen Bildung oder in der Prophylaxe des Zahnabbaus oder periodontaler Erkrankung eine orale Zusammensetzung wie definiert in einem der vorgehenden Ansprüche.